Europäisches Patentamt

European Patent Office

Office européen des brevets

(11) Publication number: **0 189 959 B1**

# EUROPEAN PATENT SPECIFICATION

(45) Date of publication of patent specification: **07.04.93**   (51) Int. Cl.5: **C12P 19/04**

(21) Application number: **86200093.2**

(22) Date of filing: **20.01.86**

(54) **Process for the preparation of polysaccharide aqueous solutions.**

(30) Priority: **01.02.85 GB 8502629**

(43) Date of publication of application:
**06.08.86 Bulletin   86/32**

(45) Publication of the grant of the patent:
**07.04.93 Bulletin   93/14**

(84) Designated Contracting States:
**AT BE CH DE FR GB IT LI LU NL SE**

(56) References cited:
**EP-A- 0 040 445**
**GB-A- 2 085 904**

(73) Proprietor: **SHELL INTERNATIONALE RE-
SEARCH MAATSCHAPPIJ B.V.**
**Carel van Bylandtlaan 30**
**NL-2596 HR Den Haag(NL)**

(72) Inventor: **Nisbet, Timothy Michael**
**15 Millfield**
**Sittingbourne Kent(GB)**
Inventor: **Linton, John Dudley**
**16 Harvey Drive**
**Sittingbourne Kent(GB)**
Inventor: **Godley, Andrew Richard**
**107 Tonge Road**
**Sittingbourne Kent(GB)**

## Description

The present invention relates to a process for the preparation of a polysaccharide aqueous solutions, more particularly aqueous solutions of polysaccharides of the succine-glucan type.

A polysaccharide of the succino-glucan type may be defined as a heteropolysaccharide comprising glucose and for each 7 moles of glucose, 0.9 to 1.2 moles of galactose and 0.65 to 1.1 moles of pyruvate, together with succinate and acetate in molar proportions (for each 7 moles of glucose) between 0 and 2. Microorganisms which produce such polysaccharides are herein referred to as "polysaccharide-producing microorganisms of the succino-glucan type".

From EP-A-40445, (Applicants ref K 1480), a process is described for the production of a polysaccharide of the succino-glucan type which comprises cultivating a slime-forming species of Pseudomonas, Rhizobium, Alcaligenes or Agrobacterium, e.g. Pseudomonas sp. NCIB 11264, Pseudomonas sp. NCIB 11592 or mutants thereof, under aerobic conditions in an aqueous nutrient medium and recovering the polysacchardie from the polysacchardie containing medium.

In EP-A-138255 (Applicant's ref K 1924), there is described an improved process for the production of a polysaccharide of the succino-glucan type, involving the cultivation of Agrobacterium radiobacter NCIB 11883. Products which are obtained from the processes of EP-A-40445 or EP-A-138255 have been found to require prefiltration if they are to pass through a cellulase acetate filter of pore diameter 1.2$\mu$m (e.g. a 1.2$\mu$m "Millipore" filter) ("Millipore" is a trademark). Ability of a polysaccharide solution to pass through such filter is a useful indication that the polysaccharide will function satisfactorily in enhanced oil recovery operations, in that the injection of the polysaccharide-containing aqueous displacement formulation into the oil- or gas-bearing rock formation will not be hindered by material which can cause plugging of the small pores of the rock formation.

US Patent 4,416,990 describes the use of Basidiomycete cellulase to treat an aqueous dispersion of a xanthan gum containing bacteria cell residues and microgels as impurities in order to improve the injectability and filterability of the xanthan gum in oil formations and thus improving the recovery of crude oil.

GB 2065688B describes a treatment of a polysaccharide with an endoenzyme capable of hydrolysing at least one of the linkages between sugar units of the polysaccharide, in order to enhance the ability of a polysaccharide in an aqueous solution to flow through a porous medium.

A process has now been found for improving injectability and filterability of polysaccharides of the succino-glucan type.

The present invention provides a process for the preparation of a polysaccharide aqueous solution, which comprises aerobically fermenting an assimilable carbohydrate source with a polysaccharide producing microorganism of the succino-glucan type in an aqueous nutrient medium characterised by adding cellulase to the fermentation broth. The amount of cellulase which is added to the fermentation broth depends, inter alia, on the level of activity of the enzyme and the concentration of microorganisms. However, in general cellulase may conveniently be added at a rate in the range 0.01 to 0.2gl$^{-1}$, preferably 0.05 to 0.2gl$^{-1}$. The term cellulase refers to any enzyme able to break down the $\beta$-1$\rightarrow$4 link of cellulose.

Examples of commercially available enzyme preparations possessing cellulase activity include "Cellulase CP" ex Sturge Ltd, "Cellulase AC" ex Miles Biochemicals, "Cellulosin AP" ex Miles Biochemicals, "Xylanase" ex Miles Biochemicals, "Celluclast" ex Novo, and cellulase ex. Gist-Brocades B.V.

Preferred suitable polysaccharide producing microorganisms comprise the group consisting of Rhizobium meliloti, Rhizobium trifolii, Alcaligenes faecalis var. myxogenes, Agrobacterium tumefaciens, Agrobacterium radiobacter, Agrobacterium rhizogenes, Pseudomonas spp. and mutants thereof. Particularly preferred are the species Agrobacterium radiobacter NCIB 11883, Pseudomonas sp. NCIB 11264, and Pseudomonas sp. NCIB 11592.

The polysaccharides products which can be obtained with the present process e.g. aqueous polysaccharide solutions, can conveniently find employment as water thickeners which are used in oil and gas exploration and production operations. Possible applications of polysaccharides in this field of technology can be expected in completion-, work-over-, drilling- and fluid displacement formulations.

However since the present process is in particular an excellent method for removing potential plugging particulates from the polysaccharide formulation, the polysaccharide aqueous solutions obtained with the present process find advantageous application (as thickeners) in aqueous fluid displacement solutions in enhanced oil recovery operations.

The present invention will be further understood from the following Examples.

Example I

A batch fermentation with Agrobacterium radiobacter strain NCIB 11883 was carried out at a pH of 7 and a temperature of 36°C in the following medium.

| | |
|---|---|
| $KH_2PO_4$ | $0.68gl^{-1}$ |
| $MgSO_4\ 7H_2O$ | $0.49gl^{-1}$ |
| $MnSO_4\ 4H_2O$ | $0.44mgl^{-1}$ |
| $ZnSO_4\ 7H_2O$ | $0.57mgl^{-1}$ |
| $H_3BO_3$ | $0.06mgl^{-1}$ |
| $Na_2MoO_4\ 2H_2O$ | $0.24mgl^{-1}$ |
| $(NH_4)_2SO_4$ | $0.79gl^{-1}$ |
| $CaCl_2\ 2H_2O$ | $7.35mgl^{-1}$ |
| $CuSO_4\ 5H_2O$ | $0.25mgl^{-1}$ |
| $CoCl_2\ 6H_2O$ | $0.23mgl^{-1}$ |
| $KI$ | $0.16mgl^{-1}$ |
| $FeSO_4\ 7H_2O$ | $13.9mgl^{-1}$ |
| Glucose | $25gl^{-1}$ |

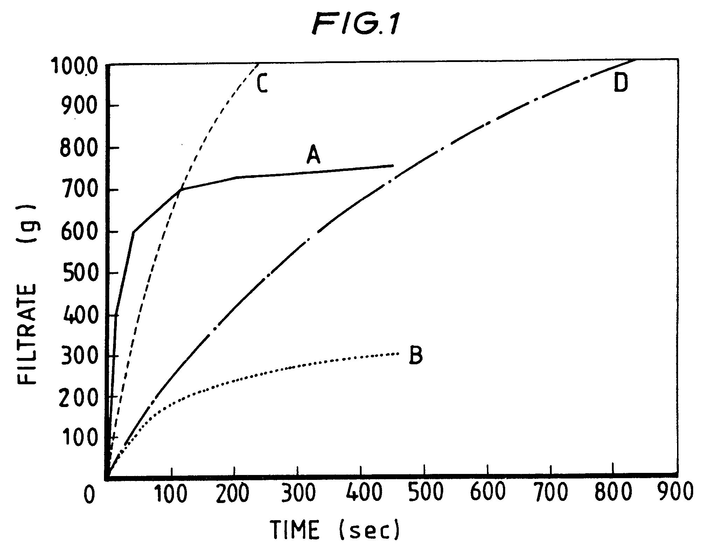

Broth samples were taken at the end of cell growth ($7\frac{1}{2}$ hours, determined by optical density measurement - end of growth marked by constant value), and at glucose exhaustion (a further 70 hours, determined by analysis of fermentation broth). A similar fermentation was carried out, except that cellulase ($0.2gl^{-1}$) was added with the inoculum of NCIB 11883. Samples were taken as above. Samples (80ml) were diluted x 13 (v/v) by addition of 12 volumes of aqueous 15% w/v NaCl + 1.5% w/v $CaCl_2$, mixed for 2 minutes (using an "Ultraturrax" (trade mark) mixer), and filterability was tested by measuring the rate of flow through a $1.2\mu m$, 47mm diameter "Millipore" (trade mark) filter, at 30°C, with 40 psi overpressure. Figure 1 gives the results. Curve A is for the untreated broth (end growth) and curve B for untreated broth (glucose exhaustion). Curve C is for the cellulase treated broth (end growth) and curve D is for the cellulase treated broth (glucose exhaustion). Filterability of the treated broth is in both cases markedly superior.

Example II

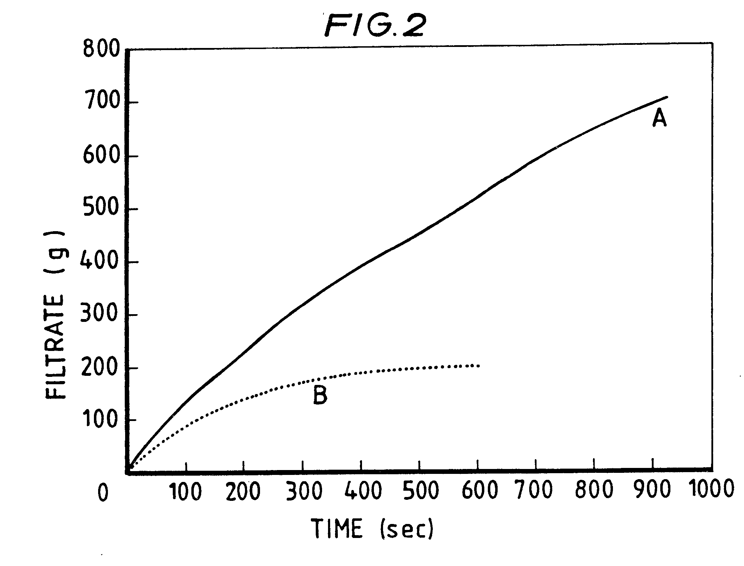

A bath fermentation with Agrobacterium radiobacter strain NCIB 11883 was carried out as in Example I. At the end of cell growth, cellulase ($0.2gl^{-1}$) was added to the broth, and the fermentation allowed to proceed to glucose exhaustion. Filterability of the broth (diluted x 10) was tested as in Example 1. Figure 2 shows the result for this broth (curve A) compared to broth where no cellulase is added (curve B). Filterability of the treated broth is markedly superior.

Example III

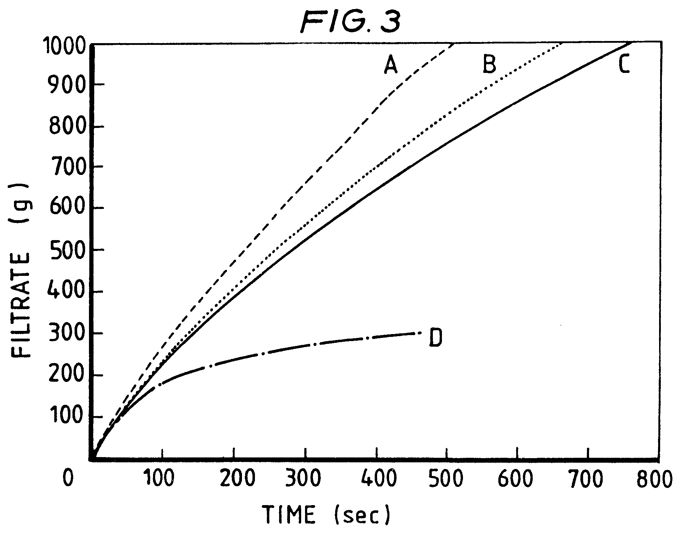

A batch fermentation with Agrobacterium radiobacter strain NCIB 11883 was carried out as in Example I. At glucose exhaustion, the broth pH was adjusted to 5.5, and cellulase was added to 0.1, 0.05 or $0.019gl^{-1}$. Broth was incubated at 40°C, and harvested after 1.8 hours. Filterability was measured as in Example I. Results are in Figure 3. Treatment with $0.1gl^{-1}$ (curve A), $0.05gl^{-1}$ (curve B) or $0.019\ gl^{-1}$ (curve C) cellulase gave a large improvement over the untreated broth (curve D).

Example IV

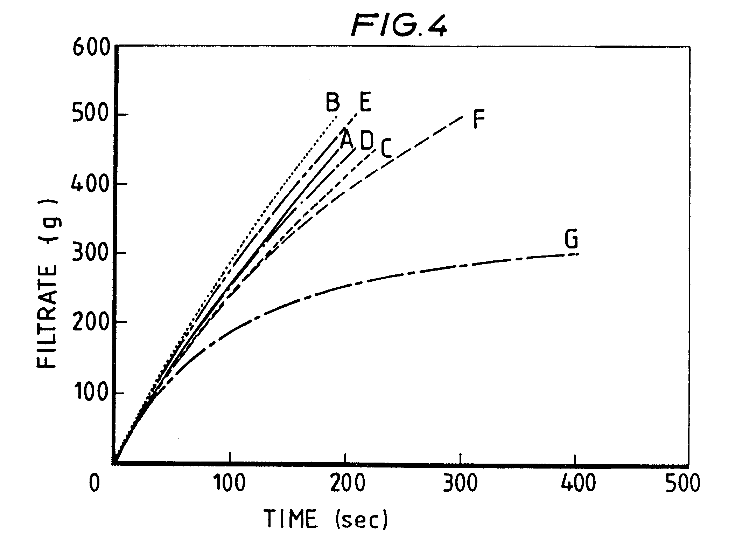

A batch fermentation with Agrobacterium radiobacter strain NCIB 11883 was carried out as in Example I. At glucose exhaustion, the broth pH was adjusted to 5.5, and one of a number of commercially available cellulase preparations was added ($0.2gl^{-1}$). After 5.75 hours incubation at 40°C, broth filterability was tested as in Example I (x 10 dilution). Results are in Figure 4. Addition of $0.2gl^{-1}$: "Cellulase CP" (Sturge Ltd. - curve A), cellulase (Gist-Brocades - curve B), "Cellulase AC" (Miles Biochemicals - curve C), "Cellulosin AP" (Miles Biochemicals - curve D), "Xylanase" (Miles Biochemicals - curve E) or $0.2ml.l^{-1}$ "Celluclast" (Novo - curve F) gave a dramatic improvement in filterability compared to the untreated broth (curve G).

Example V

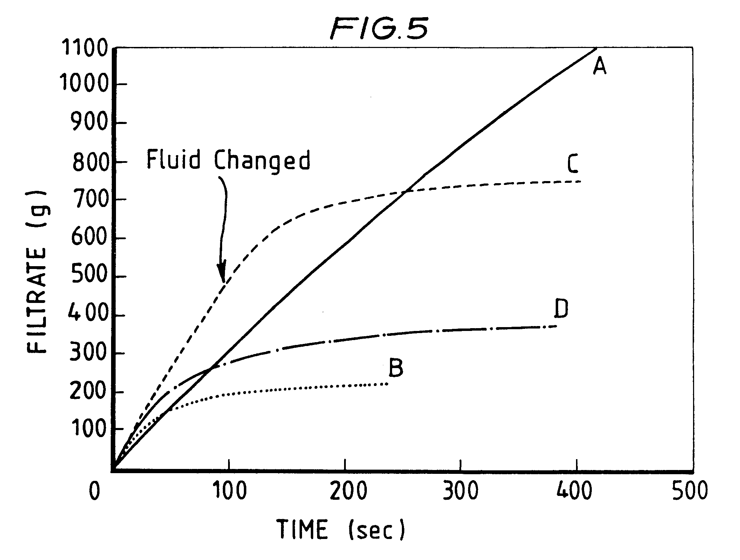

A batch fermentation with Agrobacterium radiobacter strain NCIB 11883 was carried out as in Example I. A sample of broth was taken at glucose exhaustion, and the remainder of the broth treated with cellulase ($0.2gl^{-1}$) for 7 hours at pH 5.5 and 40°C. Filterability was measured as in Example 1 (x 10 dilution). Results are given in Figure 5. Cellulase treated broth (curve A) had excellent filterability. Non-treated broth filtered poorly (curve B). If 500ml of solution prepared from cellulase treated broth was followed by non-cellulase treated solution, blocking occurred rapidly after the change of solution (curve C). If cellulase was added to a non-treated product immediately prior to filtration, blocking occurred rapidly (curve D). These results demonstrate that the above results are due to action of cellulase on the microbial broth, and not on the cellulose acetate of the "Millipore" (trademark) filter.

## Claims

1. Process for the preparation of a polysaccharide aqueous solution, which comprises aerobically fermenting an assimilable carbohydrate source with a polysaccharide producing microorganism of the succino-glucan type in an aqueous nutrient medium characterised by adding cellulase to the fermentation broth.

2. Process as claimed in claim 1, in which 0.01 to 0.2gl$^{-1}$ of cellulase is added to the fermentation broth.

3. Process as claimed in claim 2 in which 0.05 to 0.2 gl$^{-1}$ of cellulase is added to the fermentation broth.

4. Process as claimed in claim 1, 2 or 3 in which the polysaccharide producing microorganism is selected from the group consisting of Rhizobium meliloti, Rhizobium trifolii, Alcaligenes faecalis var. myxogenes, Agrobacterium tumefaciens, Agrobacterium radiobacter, Agrobacterium rhizogenes, Pseudomonas spp. and mutants thereof.

5. Process as claimed in claim 4 in which the microorganism is selected from the group consisting of Pseudomonas sp. NCIB 11264, Pseudomonas sp. NCIB 11592 and Agrobacterium radiobacter NCIB 11883.

6. Use of a polysaccharide aqueous solution obtained with a process as claimed in any one of claims 1 to 5 in an aqueous fluid displacement solution in an enhanced oil recovery operation.

## Patentansprüche

1. Verfahren zur Herstellung einer wäßrigen Polysaccharidlösung, welches ein aerobes Fermentieren einer assimilierbaren Kohlenhydratquelle mit einem Polysaccharid-bildenden Mikroorganismus vom Succino-Glucan-Typ in einem wäßrigen Nährmedium unfaßt, gekennzeichnet durch Zusetzen von Cellulase zur Fermentationsbrühe.

2. Verfahren nach Anspruch 1, worin 0,01 bis 0,2 g.l$^{-1}$ Cellulase zu der Fermentationsbrühe zugesetzt werden.

3. Verfahren nach Anspruch 2, worin 0,05 bis 0,2 g.l$^{-1}$ Cellulase zu der Fermentationsbrühe zugesetzt werden.

4. Verfahren nach Anspruch 1, 2 oder 3, worin der Polysaccharid-bildende Mikroorganismus aus der Rhizobium meliloti, Rhizobium trifolii, Alcaligenes faecalis var. myxogenes, Agrobacterium tumefaciens, Agrobacterium radiobacter, Agrobacterium rhizogenes, Pseudomonas spp. und Mutanten hievon umfassenden Gruppe ausgewählt wird.

5. Verfahren nach Anspruch 4, worin der Mikroorganismus aus der Pseudomonas sp. NCIB 11264, Pseudomonas sp. NCIB 11592 und Agrobacterium radiobacter NCIB 11883 bestehenden Gruppe ausgewählt wird.

6. Verwendung einer wäßrigen Polysaccharidlösung, erhalten nach einem Verfahren, wie in einem der Ansprüche 1 bis 5 beansprucht, in einer wäßrigen Fluidverdrängungslösung in der tertiären Ölgewinnung.

## Revendications

1. Procédé pour la préparation d'une solution aqueuse de polysaccharide, qui comprend la fermentation dans des conditions aérobies d'une source d'hydrate de carbone assimilable avec un microorganisme producteur de polysaccharide du type succino-glucanne dans un milieu nutritif aqueux, caractérisé en ce qu'on ajoute de la cellulase au bouillon de fermentation.

2. Procédé selon la revendication 1, dans lequel on ajoute de 0,01 à 0,2 gl$^{-1}$ de cellulase au bouillon de fermentation.

3. Procédé selon la revendication 2, dans lequel on ajoute de 0,05 à 0,2 gl$^{-1}$ de cellulase au bouillon de fermentation.

4. Procédé selon la revendication 1, 2 ou 3, dans lequel le microorganisme producteur de polysaccharide est choisi dans le groupe constitué par Rhizobium meliloti, Rhizobium trifolii, Alcaligenes faecalis var. myxogenes, Agrobacterium tumefaciens, Agrobacterium radiobacter, Agrobacterium rhizogenes, Pseudomonas spp. et leurs mutants.

5. Procédé selon la revendication 4, dans lequel le microorganisme est choisi dans le groupe constitué par Pseudomonas sp. NCIB 11264, Pseudomonas sp. NCIB 11592 et Agrobacterium radiobacter NCIB 11883.

6. Utilisation d'une solution aqueuse de polysaccharide obtenue avec un procédé selon l'une quelconque des revendications 1 à 5 dans une solution aqueuse de déplacement de fluides dans une opération d'extraction améliorée du pétrole.

FIG.1

FIG.2

FIG.3

FIG.4

FIG.5

Fluid Changed